# EUROPÄISCHE PATENTSCHRIFT

(11) **EP 1 429 663 B1**
(45) Veröffentlichungstag und Bekanntmachung des Hinweises auf die Patenterteilung: **27.04.2005**
(21) Anmeldenummer: 02800084.2
(22) Anmeldetag: 13.09.2002
(51) Int. Cl.: A61B 17/04, A61B 17/17

(54) **VORRICHTUNG ZUM EINBRINGEN EINES FADENANKERS IN EINEN KNOCHEN**
DEVICE FOR INTRODUCING A THREAD ANCHOR INTO A BONE
DISPOSITIF POUR INTRODUIRE UN ELEMENT D'ANCRAGE DE FIL DANS UN OS

(30) Priorität: 26.09.2001 DE 10149396
(43) Veröffentlichungstag der Anmeldung: 23.06.2004
(73) Patentinhaber: Karl Storz GmbH & Co. KG, 78532 Tuttlingen (DE)
(72) Erfinder: TIMMERMANS, André, NL-7261 JH Ruurlo (NL); LAJTAI, Georg, A-9330 Althofen (AT)
(74) Vertreter: Weller, Wolfgang, Dr.
(86) Internationale Anmeldenummer: PCT/EP2002/010298
(87) Internationale Veröffentlichungsnummer: WO 2003/028559

(56) Entgegenhaltungen:
- WO-A-96/15727
- DE-U- 20 105 590
- US-A- 5 584 839
- US-A- 5 690 677
- US-A- 5 938 686

## Beschreibung

Die Erfindung betrifft eine Vorrichtung zum Einbringen eines Fadenankers in einen Knochen, mit einer hohlen Führungshülse, durch die der Fadenanker hindurchbringbar ist, wobei am distalen Ende der Führungshülse eine Konturierung vorgesehen ist, die ein Positionieren des distalen Endes erleichtert.

Eine derartige Vorrichtung ist aus der US 5,951,559 oder aus der DE-A-20 105 580 bekannt.

Ein Fadenanker wird in einen Knochen eingesetzt, um ein von diesem Knochen abgelöstes Gewebe, sei es eine Sehne oder ein Knorpel oder ein Knochenteil, wieder zu befestigen. Der Fadenanker wird in den Knochen eingetrieben, wobei am proximalen Ende des Fadenankers eine Öse vorhanden ist, durch die ein Faden gefädelt ist. Nach dem Einbringen des Fadenankers in den Knochen wird die vom Knochen abgelöste Sehne durch Verknoten des Fadens wieder an den Knochen herangebracht bzw. angelegt.

Ein typisches Kranheitsbild, bei dem solche Fadenanker eingesetzt werden, sind Schulterverletzungen bzw. -instabilitäten.

Die Gelenkpfanne (Glenoid), in der der Kopf (Humeruskopf) des Oberarmes sitzt, ist von einem kraterartig hochstehenden Rand von Knorpel und Sehnengewebe umrundet. Mit diesem Glenoidrand ist auch die Sehne des Oberarmmuskels (die Bizepssehne) verbunden.

Bei einer sogenannten Bankart-Läsion wird die traumatische Ablösung des oberen Labrumanteils unter Einbeziehung des Ansatzes der langen Bizepssehne vom Glenoidrand verstanden.

Es sind dazu verschiedene Unfallmechanismen bekannt. Durch extremen Zug an der langen Bizepssehne, z.B. am Ende einer Wurfphase, kann es beim Abbremsvorgang bei gleichzeitig maximal gestrecktem Ellenbogen zur Labrumsläsion kommen. Auch Stürze auf den Ellenbogen bei gebeugtem Arm mit Subluxation des Humeruskopfes nach superior können den oberen Labrumanteil und die lange Bizepssehne nach proximal abschieben.

Bei der eingangs genannten Operationstechnik werden nun die vom Glenoidrand abgelösten Knorpel oder ein Knochenteil oder Sehnenteile dadurch wieder fixiert, daß ein Fadenanker eingebracht wird, mittels dessen Faden die Sehne wieder an Ort und Stelle fixiert wird.

Bei der in der eingangs genannten US 5,951,559 beschriebenen Operationstechnik ist ersichtlich, daß der Fadenanker am distalen Ende eines Fadendrehers, einer Art Schraubendreher, eingesetzt wird und durch eine hohle Führungshülse hindurchgeschoben wird. Bei der eigentlichen Operation wird diese hohle Führungshülse durch ein arthroskopisches Portal in das Schultergelenk eingeführt und am Glenoidrand angesetzt. Zum Erleichtern und sicheren Positionieren ist dazu am distalen Ende der Führungshülse eine Konturierung vorgesehen.

Bei der eingangs genannten US 5,951,559 besteht diese Konturierung aus einer Kontur in der Art einer zweizinkigen Gabel, die so ausgebildet ist, daß sie passend und fest sitzend auf den kraterrandartig hochstehenden Glenoidrand aufgesetzt werden kann. Durch die derart aufgesetzte Führungshülse können dann die notwendigen Manipulationen durchgeführt werden. Es kann also zunächst einmal ein Bohrer hindurchgeführt werden, um die Bohrlöcher für den Anker zu fertigen, anschließend wird der Zusammenbau aus Fadenanker und Fadendreher hindurchgebracht und der Anker eingedreht. Anschließend wird mit weiteren Instrumenten der vom Anker nach proximal vorstehende Faden um den abgelösten Glenoidrand herumgefädelt und mit diesem verknotet, um diesen wieder am Glenoid zu befestigen.

Aus der US 5,690,677 ist es auch schon bekannt geworden, die Führungshülse zusätzlich noch abzuschrägen, so daß eine messeroder skalpellartige Spitze entsteht. Zusätzlich ist diese Spitze, etwa um 90° um die Längsachse verdreht, noch mit dem gabelartigen Vorsprung versehen, um auf dem Glenoidrand anzusetzen.

Nachteilig an den vorgenannten Konstruktionen ist, daß zwar die Hülse sicher sitzend auf dem Glenoidrand angesetzt werden kann, daß es aber manchmal aufgrund der Läsion oder aufgrund anatomischer Gegebenheiten wünschenswert ist, den Fadenanker nicht direkt unterhalb des Glenoidrands einzusetzen sondern seitlich versetzt oder in gekippter Richtung.

Es ist daher Aufgabe der vorliegenden Erfindung, eine Vorrichtung der eingangs genannten Art dahingehend weiterzuentwickeln, daß ein Fadenanker in zahlreichen, auch bezüglich eines Glenoidrands versetzten oder gekippten Positionen eingebracht werden kann, dennoch aber ein sicherer Sitz der Führungshülse gewährleistet ist.

Erfindungsgemäß wird die Aufgabe dadurch gelöst, daß die Konturierung als eine von einer stirnseitigen Ringfläche der Führungshülse distal vorspringende Spitze ausgebildet ist, und daß die verbleibende Ringfläche mit der Spitze eine Schulter bildet.

Durch das Vorsehen einer Schulter und nur einer Spitze ist es nunmehr möglich, das distale Ende der Führungshülse sehr variabel, aber dennoch sicher sitzend anzusetzen. Daraus resultiert ein erheblicher Freiheitsgradzugewinn an Positionierungsmöglichkeiten, d.h. eine angesetzte Führungshülse kann in mehreren Richtungen gekippt werden, wobei die Schulter zwischen Ringfläche und Spitze dennoch einen sicheren Sitz gewährleistet.

Je nachdem, wie die Spitze ausgebildet ist, kann diese auch dazu dienen, zunächst einmal durch Einstechen der Spitze in das Knorpel- oder Sehnengewebe einen vorläufigen Ansatzpunkt zu suchen und danach, gegebenenfalls unter endoskopischer Beobachtung, eine zutreffende Ausrichtung oder Orientierung zu wählen. Ist dies erreicht, kann dann die Führungshülse so weit eingeschoben werden, bis diese über deren Schulter anliegt und dadurch für den sicheren Sitz sorgt. Es haben sich also die Manipulationsmöglichkeiten erhöht, wobei ein sicherer Sitz beibehalten wird.

In einer weiteren Ausgestaltung der Erfindung erstreckt sich die Ringfläche etwa senkrecht zur Längsachse der Führungshülse.

Diese Maßnahme hat den Vorteil, daß bei dieser Geometrie dem Operateur zusätzliche Orientierungshilfen bzw. Ansatzhilfen gegeben werden und, je nach Ausgestaltung der Spitze, eine mehr oder weniger scharf ausgeprägte oder großräumige Schulter vorhanden ist, um die Führungshülse zu positionieren.

In einer weiteren Ausgestaltung der Erfindung ist die Spitze als von der Ringfläche vorspringende nadelartige Spitze ausgebildet.

Diese Maßnahme hat den Vorteil, daß mit einer solchen nadelartigen Spitze zunächst einmal eingestochen werden kann, um eine erste Position zu erzielen, daß dann aber die Führungshülse noch in eine andere Position gekippt werden kann und erst dann der Fadenanker gesetzt wird.

In einer weiteren Ausgestaltung der Erfindung weist die nadelartige Spitze einen äußersten Spitzpunkt auf, der auf einer äußeren Mantellinie der Führungshülse liegt.

Diese Ausgestaltung hat den Vorteile daß der Spitzpunkt an dem äußersten Rand der Führungshülse zum Liegen kommt, so daß ein möglichst großer Manipulationsraum innerhalb des Umfangkreises der Führungshülse zur Verfügung bleibt.

In einer weiteren Ausgestaltung der Erfindung springt die Spitze über einen Umfangsbereich der stirnseitigen Ringfläche vor.

Diese Maßnahme hat den Vorteil, daß, je größer der Umfangsbereich ist, die Spitze um so mehr als zusätzliche Anlagefläche dienen kann.

In einer weiteren Ausgestaltung der Erfindung beträgt der Umfangsbereich etwa bis zu dem halben Umfang der Ringfläche.

Diese Maßnahme hat den Vorteil, daß dadurch eine relativ großflächige seitliche Anlage im Bereich der Spitze geschaffen werden kann, zugleich aber zumindest ein halber Umfang der gesamten Ringfläche als Fläche zur Bildung der Schulter zur Verfügung steht.

In einer weiteren Ausgestaltung der Erfindung weist die vorspringende Spitze eine bogenförmig gekrümmte Kontur auf.

Diese Maßnahme hat den Vorteil, daß in bestimmte Raum- bzw. Verschwenkrichtungen eine dauerhaft große Anlagefläche zur Verfügung bleibt, die durch die sanfte Kontur atraumatisch an den Gewebeflächen entlanggleitet, an denen die Spitze angelegt ist.

Diese Ausgestaltung kann auch dazu genützt werden, um eine relativ großflächige Schneidefläche zur Verfügung zu haben, so daß sich das distale Ende der Führungshülse schneidend vorarbeiten kann, um beispielsweise an einer unstrukturierten, unkontrollierten Knorpelfläche einen entsprechenden Sitz anzuschneiden.

In einer weiteren Ausgestaltung der Erfindung ist die Spitze durch einen Schrägschnitt am distalen Ende der Führungshülse gebildet.

Diese Maßnahme hat den Vorteil, daß nicht nur die gewünschte Geometrie einfach herstellbar ist, sondern daß sanfte Gleitund Anlageflächen geschaffen sind.

In einer weiteren Ausgestaltung der Erfindung ist ein Ankerdreher vorgesehen, dessen Schaft durch die Führungshülse schiebbar ist.

Diese an sich bekannte Maßnahme vervollständigt das Instrumentarium zur Durchführung solcher Operationen.

In einer weiteren Ausgestaltung der Erfindung entspricht der Außendurchmesser des Schaftes des Ankerdrehers dem lichten Innendurchmesser der Führungshülse.

Diese Maßnahme trägt vorteilhafterweise dazu bei, eine gute und gezielte Führung beim Setzen des Fadenankers sicherzustellen.

In einer weiteren Ausgestaltung der Erfindung ist ein Fadenanker im Ankerdreher eingesetzt.

Diese Maßnahme hat den Vorteil, daß ein aufeinander abgestimmtes Instrumentarium zum Durchführen des Setzens des Fadenankers bereitgestellt wird.

Es versteht sich, daß die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Ausführungsbeispiele der Erfindung werden nachfolgend anhand der beiliegenden Zeichnungen näher beschrieben und erläutert. Es zeigen:
- Fig. 1: einen Längsschnitt eines Ankerdrehers,
- Fig. 2: einen stark vergrößerten Schnitt des distalen Endes des Ankerdrehers, in den ein Fadenanker eingesetzt werden soll,
- Fig. 3: einen entsprechenden Fadenanker zum Einsetzen in das distale Ende des Ankerdrehers,
- Fig. 4: ein erstes Ausführungsbeispiel einer erfindungsgemäßen Führungshülse mit einer Spitze, die sich etwa um den halben Umfang der stirnseitigen Ringfläche weg erstreckt, wobei der in Fig. 1 gezeigte Ankerdreher passend durch die Führungshülse hindurchgeschoben werden kann,
- Fig. 5: eine Seitenansicht des distalen Endbereiches der Führungshülse von Fig. 4,
- Fig. 6: einen Schnitt längs der Linie VI-VI in Fig. 5,
- Fig. 7: einen Längsschnitt durch eine erfindungsgemäße Vorrichtung im Bereich des distalen Endbereiches, d.h. der Führungshülse von Fig. 4, in die ein Ankerdreher von Fig. 1 eingeschoben ist, in dessen distalen Endbereich der in Fig. 3 dargestellte Anker eingesetzt ist, wobei hier noch zusätzlich der Faden angedeutet ist, wobei diese Vorrichtung während einer Fixation des Labrum-Bandkomplexes auf einem Glenoidrand angesetzt ist,
- Fig. 8: eine der Fig. 4 entsprechende Darstellung eines weiteren Ausführungsbeispiels einer Führungshülse mit einer nadelartig vorstehenden Spitze,
- Fig. 9: eine der Fig. 5 entsprechende Darstellung einer Seitenansicht, und
- Fig. 10: einen der Fig. 6 entsprechenden Schnitt dieses Ausführungsbeispiels einer Führungshülse,
- Fig. 11: eine der Darstellung von Fig. 7 entsprechende Ansicht, wobei auch hier in das Ausführungsbeispiel einer Führungshülse mit nadelartiger Spitze der Ankerdreher von Fig. 1 mit darin eingesetztem Fadenanker von Fig. 3 dargestellt ist.

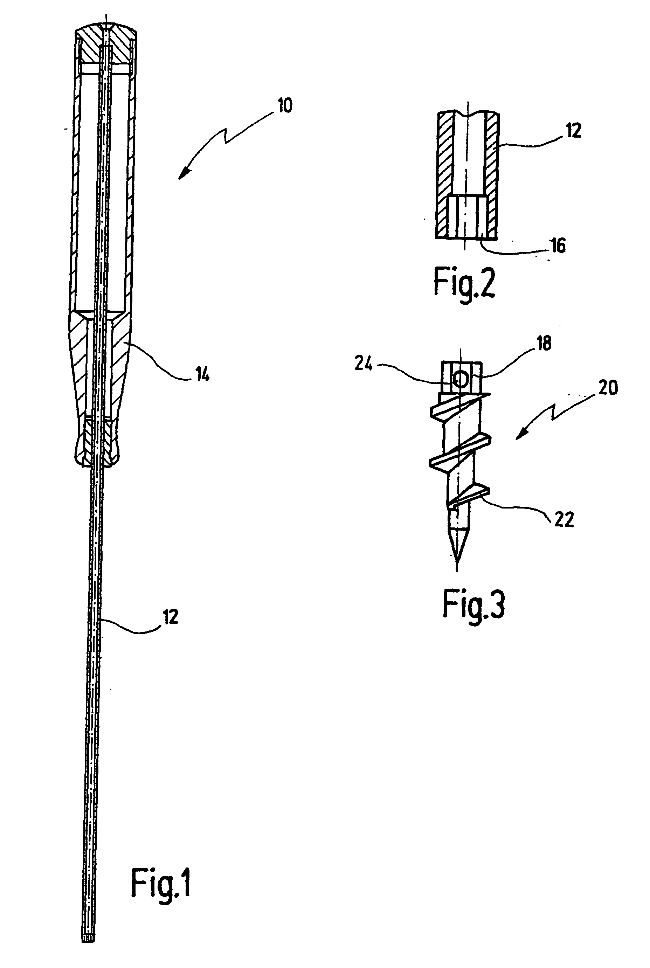

Ein in Fig. 1 dargestellter Ankerdreher 10 weist einen langerstreckten Schaft 12 und einen Griff 14 auf.

Am distalen Ende, wie es in Fig. 2 vergrößert dargestellt ist, ist der Schaft 12 mit einem Innensechskant 16 versehen, in den passend ein Außensechskant 18 eines Fadenankers 20 eingesetzt werden kann, wie er in Fig. 3 dargestellt ist.

Der Fadenanker 20 weist an einer Außenseite ein Gewinde 22 auf und ist an seinem proximalen Ende mit einer Öse 24 versehen, durch die ein hier nicht dargestellter Faden hindurchgezogen werden kann.

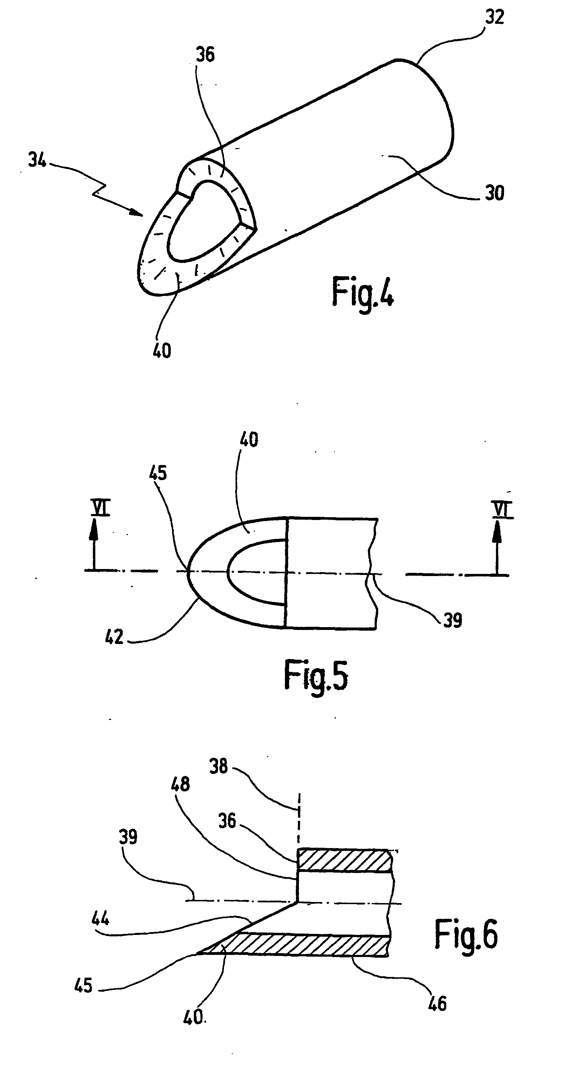

In Fig. 4 ist ein erstes Ausführungsbeispiel einer Führungshülse 30 dargestellt, und zwar lediglich im Bereich deren distalem Ende.

Die Führungshülse 30 besteht aus einem Rohr 32, das an seinem proximalen Ende mit einem seitlich abstehenden Handgriff (hier der Übersichtlichkeit halber nicht dargestellt) versehen ist. Das distale Ende 34 weist eine stirnseitige Ringfläche 36 auf, deren Ebene 38 etwa senkrecht zur Längsachse 39 der Führungshülse 30 verläuft, wie das insbesondere aus den Fig. 5 und 6 ersichtlich ist.

Von der stirnseitigen Ringfläche 36 springt eine Spitze 40 vor, die eine etwa bogenförmige Kontur 42 aufweist. Die umfängliche Erstreckung der Spitze 40 ist so, daß sie etwa den halben Umfang der stirnseitigen Ringfläche 36 einnimmt.

Wie insbesondere aus Fig. 6 zu entnehmen ist, ist die Spitze so entstanden, daß ein Schrägschnitt 44 angebracht worden ist.

Der äußerste Spitzpunkt 45 liegt dabei auf einer äußeren Mantellinie 46 des Rohres 32. Der Schrägschnitt 44 ist so angeführt, daß er etwa einen Winkel von 30° zur Längsachse 39 einschließt. Der Winkel des Schrägschnittes 44 und auch die umfängliche Erstreckung der Spitze 40 kann variieren, es bleibt aber immer sichergestellt, daß zwischen der Spitze 40 und der stirnseitigen Ringfläche 36 eine Schulter 48 ausgebildet ist.

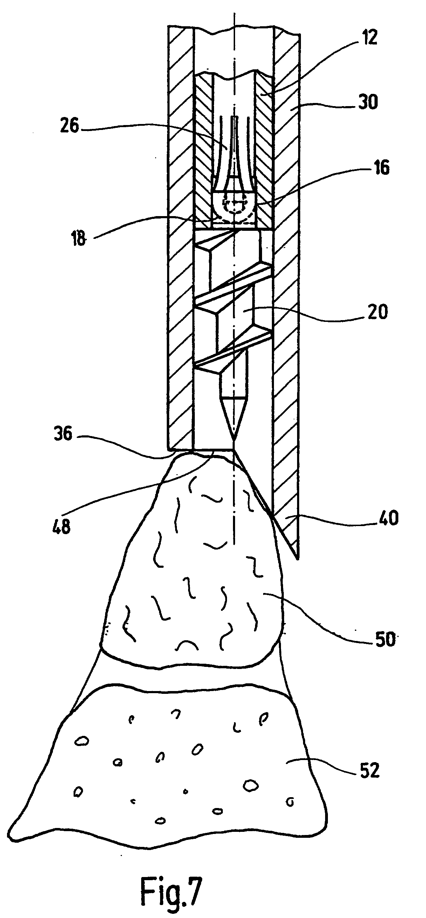

In Fig. 7 ist dargestellt, wie die erfindungsgemäße Vorrichtung beim Setzen eines Fadenankers 20 eingesetzt wird, der dazu dient, um einen aus Knorpelgewebe und Sehnen bestehenden Glenoidrand 50, der sich von dem Knochen 52 des Glenoidrandes 50 gelöst hat, wieder zu fixieren. In Fig. 7 ist eine Situation dargestellt, wie der Zusammenbau aus Führungshülse 30, Ankerdreher 10 und Fadenanker 20 nebst Faden 26 an dem Glenoidrand 50 angesetzt ist. Durch die Schulter 48 kann dieser Zusammenbau sicher sitzend angesetzt werden, die gesamte Vorrichtung kann aber dennoch positioniert werden, beispielsweise durch Verschwenken nach links und rechts in der Zeichenebene oder durch Verschwenken auf den Betrachter dieser Zeichenebene zu oder von diesem weg gerichtet. Dadurch kann eine ideale Position gefunden werden, um den Fadenanker 20 zu positionieren.

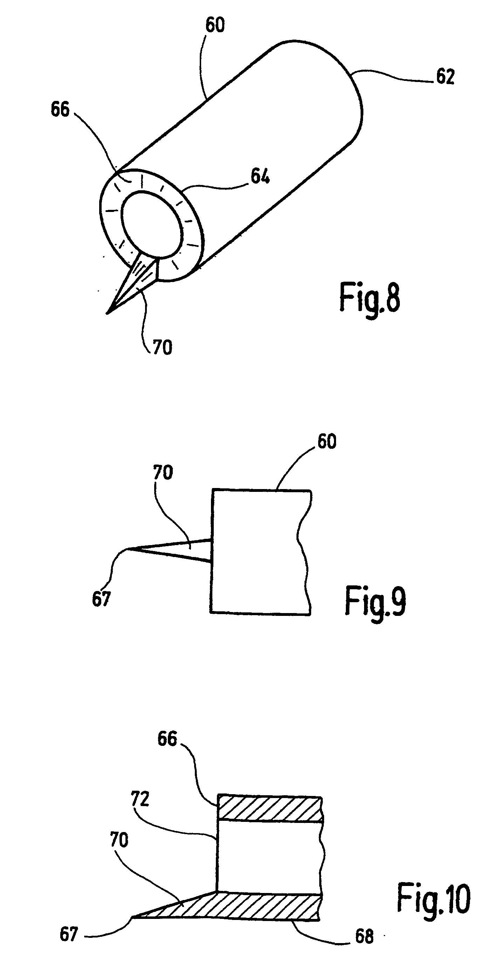

Bei einem in den Fig. 8 bis 11 dargestellten weiteren Ausführungsbeispiel besteht eine Führungshülse 60 ebenfalls aus einem Rohr 62, an dessen distalem Ende 64 eine stirnseitige Ringfläche 66 ausgebildet ist, die ebenfalls, wie zuvor beschrieben, etwa in einem rechten Winkel zur Längsachse verläuft.

Von der stirnseitigen Ringfläche 66 springt eine nadelartige Spitze 70 vor, deren äußerster Spitzpunkt 67 längs einer Mantellinie 68 des Rohres 62 liegt.

Auch hier ist wieder eine Schulter 72 zwischen der Spitze 70 und dem distalen Ende 64 geschaffen, wobei hier ein relativ großer Umfangsanteil der stirnseitigen Ringfläche 66 als Anlagefläche zur Verfügung steht.

Die Spitze 70 hat eine etwa pyramidenförmige Form, wobei sich eine äußere Fläche längs der äußeren Mantelfläche des Rohres 62 erstreckt.

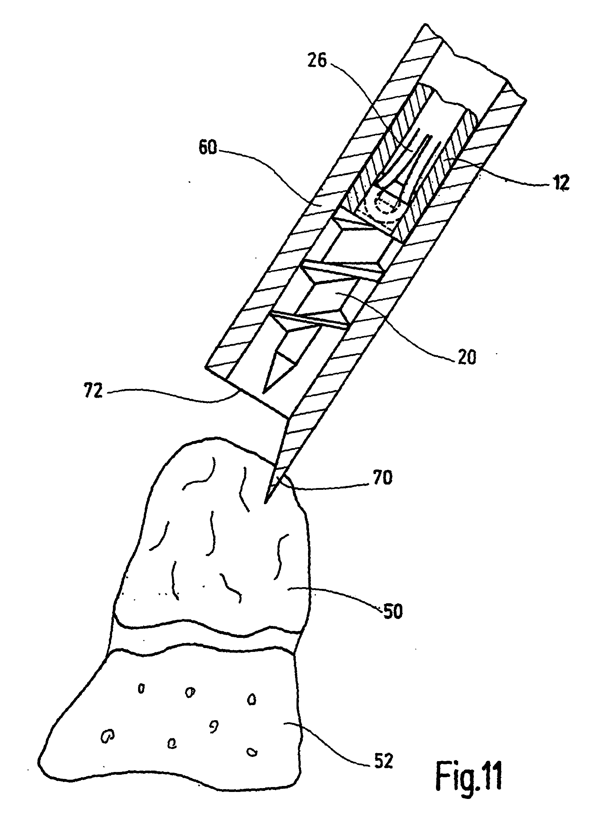

In Fig. 11 ist eine Situation dargestellt, in der in die Führungshülse 60 wiederum der Ankerdreher 10 samt darin eingesetztem Fadenanker 20 und eingefädeltem Faden 26 eingeschoben ist.

Hier kann zunächst die nadelartige Spitze 70 in das entsprechende Gewebe, beispielsweise eine Sehne oder einen Knorpel oder auch in einen Knochen, eingestochen und angesetzt werden und anschließend dann die exakte Ausrichtung gewählt werden. Durch weiteres Einschieben kommt dann wiederum die Vorrichtung über die Schulter 72 zwischen Spitze 70 und stirnseitiger Ringfläche 66 sicher zum Liegen.

## Patentansprüche

1. Rohrförmige hohle Führungshülse zum Einbringen eines Fadenankers (20) in einen Knochen (52), mit einem Rohr, (32, 62), durch das der Fadenanker (20) hindurchbringbar ist, wobei am distalen Ende (34, 64) der Führungshülse (30, 60) eine Konturierung vorgesehen ist, die ein Positionieren des distalen Endes (34, 64) erleichtert, **dadurch gekennzeichnet, dass** die Konturierung als eine von einer stirnseitigen Ringfläche (36, 66) der Führungshülse (30, 60) distal vorspringende Spitze (40, 70) ausgebildet ist, und dass die verbleibende Ringfläche (36, 66) mit der Spitze (40, 70) eine Schulter (48, 72) bildet.

2. Führungshülse nach Anspruch 1, **dadurch gekennzeichnet, dass** die Ringfläche (36, 66) sich etwa senkrecht zur Längsachse (39) der Führungshülse (30) erstreckt.

3. Führungshülse nach Anspruch 1 oder 2, **dadurch gekennzeichnet, dass** die Spitze (70) als von der Ringfläche (66) vorspringende nadelartige Spitze (70) ausgebildet ist.

4. Führungshülse nach Anspruch 3, **dadurch gekennzeichnet, dass** die nadelartige Spitze (70) einen äußersten Spitzpunkt (67) aufweist, der auf einer äußeren Mantellinie (68) der Führungshülse (60) liegt.

5. Führungshülse nach Anspruch 1 oder 2, **dadurch gekennzeichnet, dass** die vorspringende Spitze (40) über einen Umfangsbereich der stirnseitigen Ringfläche (36) vorspringt.

6. Führungshülse nach Anspruch 5, **dadurch gekennzeichnet, dass** der Umfangsbereich etwa bis zu dem halben Umfang der Ringfläche (36) beträgt.

7. Führungshülse nach Anspruch 5 oder 6, **dadurch gekennzeichnet, dass** die vorspringende Spitze (40) eine bogenförmig gekrümmte Kontur (42) aufweist.

8. Führungshülse nach einem der Ansprüche 5 bis 7, **dadurch gekennzeichnet, dass** die Spitze (40) durch einen Schrägschnitt (44) am distalen Ende (34) der Führungshülse (30) gebildet ist.

9. Führungshülse nach einem der Ansprüche 1 bis 8, **dadurch gekennzeichnet, dass** ein Ankerdreher (10) vorgesehen ist, dessen Schaft (12) durch die Führungshülse (30, 60) hindurchführbar ist.

10. Führungshülse nach Anspruch 9, **dadurch gekennzeichnet, dass** der Außendurchmesser des Schaftes (12) des Ankerdrehers (10) in etwa dem lichten Innendurchmesser der Führungshülse (30, 60) entspricht.

11. Führungshülse nach Anspruch 9 oder 10, **dadurch gekennzeichnet, dass** ein Fadenanker (20) im Ankerdreher (10) eingesetzt ist.

## Claims

1. Tubular hollow guide sleeve for introducing a thread anchor (20) into a bone (52), having a tube (32, 62) through which the thread anchor (20) can be passed, the distal end (34, 64) of the guide sleeve (30, 16) being provided with a contour which makes positioning of the distal end (34, 64) easier, **characterized in that** the contour is designed as a point (40, 70) projecting distally from a from a front annular face (36, 66) of the guide sleeve (30, 60), and **in that** the rest of the annular face (36, 66) forms a shoulder (48, 62) with the point (40, 70).

2. Guide sleeve of claim 1, **characterized in that** the annular face (36, 66) extends approximately perpendicular to the longitudinal axis (39) of the guide sleeve (30).

3. Guide sleeve of claims 1 or 2, **characterized in that** the point (70) is designed as a needle-like point (70) projecting from the annular face (66).

4. Guide sleeve of claim 3, **characterized in that** the needle-like point (70) has an outermost tip (67) which lies on an outer surface line (68) of the guide sleeve (60).

5. Guide sleeve of claims 1 or 2, **characterized in that** the projecting point (40) projects from over a circumferential area of the front annular face (36).

6. Guide sleeve of claim 5, **characterized in that** the circumferential area is about up to half the circumference of the annular face (36).

7. Guide sleeve of claims 5 or 6, **characterized in that** the projecting point (40) has a contour (42) curved in an arc shape.

8. Guide sleeve of anyone of claims 5 through 7, **characterized in that** the point (40) is formed by a beveled cut (44) at the distal end (34) of the guide sleeve (30).

9. Guide sleeve of anyone of claims 1 through 8, **characterized in that** an anchor rotator (10) is provided, whose shank (12) can be guided through the guide sleeve (30, 60).

10. Guide sleeve of claim 9, **characterized in that** the outer diameter of the shank (12) of the anchor rotator (10) corresponds approximately the clear inner diameter of the guide sleeve (30, 60).

11. Guide sleeve of claims 9 or 10, **characterized in that** a thread anchor (20) is fitted in the anchor rotator (10).

## Revendications

1. Gaine de guidage creuse tubulaire destinée à introduire une ancre pour fil (20) dans un os (52), comportant un tube (32, 62) à travers lequel peut être acheminée l'ancre pour fil (20), l'extrémité distale (34, 64) de la gaine de guidage (30, 60) ayant un contour profilé qui permet un positionnement de l'extrémité distale (34, 64), **caractérisée en ce que** le contour profilé est conçu sous forme de pointe (40, 70) en saillie distale sur une surface annulaire (36, 66) frontale de la gaine de guidage (30, 60), et **en ce que** la surface annulaire (36, 66) restante forme avec la pointe (40, 70) un épaulement (48, 72).

2. Gaine de guidage selon la revendication 1, **caractérisée en ce que** la surface annulaire (36, 66) s'étend pratiquement perpendiculairement à l'axe longitudinal (39) de la gaine de guidage (30).

3. Gaine de guidage selon la revendication 1 ou 2, **caractérisée en ce que** la pointe (70) est conçue sous forme de pointe de type aiguille (70) s'avançant en saillie sur la surface annulaire (66).

4. Gaine de guidage selon la revendication 3, **caractérisée en ce que** la pointe de type aiguille (70) comporte une extrémité extérieure (67) qui est située sur une ligne d'enveloppe extérieure (68) de la gaine de guidage (60).

5. Gaine de guidage selon la revendication 1 ou 2, **caractérisée en ce que** la pointe (40) en saillie s'avance en saillie sur une zone périphérique de la surface annulaire (36) frontale.

6. Gaine de guidage selon la revendication 5, **caractérisée en ce que** la zone périphérique représente environ jusqu'à la moitié du pourtour de la surface annulaire (36).

7. Gaine de guidage selon la revendication 5 ou 6, **caractérisée en ce que** la pointe (40) en saillie présente un contour (42) courbé en forme d'arc.

8. Gaine de guidage selon l'une quelconque des revendications 5 à 7, **caractérisée en ce que** la pointe (40) est formée par une coupe en biseau (44) au niveau de l'extrémité distale (34) de la gaine de guidage (30).

9. Gaine de guidage selon l'une quelconque des revendications 1 à 8, **caractérisée en ce qu'**il est prévu un tourne-ancre (10), dont la tige (12) est destinée à être guidée à travers la gaine de guidage (30, 60).

10. Gaine de guidage selon la revendication 9, **caractérisée en ce que** le diamètre extérieur de la tige (12) du tourne-ancre (10) correspond pratiquement au diamètre intérieur libre de la gaine de guidage (30, 60).

11. Gaine de guidage selon la revendication 9 ou 10, **caractérisée en ce qu'**une ancre pour fil (20) est insérée dans le tourne-ancre (10).
